# EUROPEAN PATENT SPECIFICATION

(11) **EP 2 278 889 B1**
(45) Date of publication and mention of the grant of the patent: **09.04.2014**
(21) Application number: 09755074.3
(22) Date of filing: 29.05.2009
(51) Int. Cl.: A23K 1/12, A23K 1/14, A23K 3/03

(54) **METHOD FOR TREATING VEGETABLE MATERIAL WITH ACID AS WELL AS PRODUCTS OBTAINED WITH THIS METHOD**
VERFAHREN ZUR BEHANDLUNG EINES PFLANZLICHEN MATERIALS MIT SÄURE SOWIE IN DIESEM VERFAHREN HERGESTELLTE PRODUKTE
PROCÉDÉ POUR TRAITER UN MATÉRIAU VÉGÉTAL AVEC UN ACIDE ET PRODUITS OBTENUS AVEC CE PROCÉDÉ

(30) Priority: 29.05.2008 NL 1035493
(43) Date of publication of application: 02.02.2011
(73) Proprietor: Agrifirm Group B.V., 7324 AE Apeldoorn (NL)
(72) Inventor: SANDERS, Johan, Pieter, Marinus, NL-9722 WL Groningen (NL); DE BOT, Petrus, Hubertus, Marie, NL-5467 KR Veghel (NL); KOOTSTRA, Anne, Maarten, Joost, NL-6721 TS Bennekom (NL)
(74) Representative: Algemeen Octrooi- en Merkenbureau B.V.
(86) International application number: PCT/NL2009/000125
(87) International publication number: WO 2009/145617

(56) References cited:
- US-A1- 2005 271 770
- LU YULIN ET AL: "Biomimetic catalysis for hemicellulose hydrolysis in corn stover" BIOTECHNOLOGY PROGRESS, vol. 23, no. 1, January 2007 (2007-01), pages 116-123, XP002512196 ISSN: 8756-7938
- SAHA B C ET AL: "Dilute acid pretreatment, enzymatic saccharification and fermentation of wheat straw to ethanol" PROCESS BIOCHEMISTRY, ELSEVIER, NL, vol. 40, no. 12, 1 December 2005 (2005-12-01), pages 3693-3700, XP025306655 ISSN: 1359-5113 [retrieved on 2005-12-01]
- KARIMI K ET AL: "Conversion of rice straw to sugars by dilute-acid hydrolysis" BIOMASS AND BIOENERGY, PERGAMON, OXFORD, GB, vol. 30, no. 3, 1 March 2006 (2006-03-01), pages 247-253, XP025109448 ISSN: 0961-9534 [retrieved on 2006-03-01]
- LLOYD T A ET AL: "Combined sugar yields for dilute sulfuric acid pretreatment of corn stover followed by enzymatic hydrolysis of the remaining solids" BIORESOURCE TECHNOLOGY, ELSEVIER, GB, vol. 96, no. 18, 1 December 2005 (2005-12-01), pages 1967-1977, XP025313205 ISSN: 0960-8524 [retrieved on 2005-12-01] cited in the application
- MOSIER N ET AL: "Features of promising technologies for pretreatment of lignocellulosic biomass" BIORESOURCE TECHNOLOGY, ELSEVIER, GB, vol. 96, no. 6, 1 April 2005 (2005-04-01), pages 673-686, XP025313257 ISSN: 0960-8524 [retrieved on 2005-04-01]
- SUN Y ET AL: "Dilute acid pretreatment of rye straw and bermudagrass for ethanol production" BIORESOURCE TECHNOLOGY, ELSEVIER, GB, vol. 96, no. 14, 1 September 2005 (2005-09-01), pages 1599-1606, XP025313364 ISSN: 0960-8524 [retrieved on 2005-09-01]
- DIEN B S ET AL: "Chemical composition and response to dilute-acid pretreatment and enzymatic saccharification of alfalfa, reed canarygrass, and switchgrass" BIOMASS AND BIOENERGY, PERGAMON, OXFORD, GB, vol. 30, no. 10, 1 October 2006 (2006-10-01), pages 880-891, XP025109390 ISSN: 0961-9534 [retrieved on 2006-10-01]

## Description

The present invention relates to a method for treating vegetable material with acid. The invention further relates to a method for enzymatic treatment of carbohydrate-containing vegetable materials.

Such a method for treating vegetable material with acid is known from Lloyd et al. (Bioresource Technology 2005, 96, 1967-1977). Lloyd et al. describe a method in which corn stalks and leaves are treated with sulphuric acid while being heated, followed by an enzymatic treatment so as to obtain sugars.

A drawback of said method is the fact that a large amount of byproducts are formed.

Mosier et al. (Biotechnology Progress 2001, 17, 474-480) describe a method in which very fine suspended cellulose is hydrolysed with sulphuric acid or carbonic acid or maleic acid. This example concerns very pure cellulose, without any objectionable additional components.

A drawback of said method for the treatment of vegetable materials is the fact that the cellulose has to be very finely distributed first and that objectionable additional compounds, such as other carbohydrate fractions and lignin have to be removed. In addition, a large amount of byproducts are formed when sulphuric acid is used.

Lu, Y. and Mosier, N. S in Biomimetic Catalysis for Hemicellulose Hydrolysis in Corn Stover. Biotechnology Progress, volume 23, pages 116-123 describe developments in the use of a dicarboxylic acid catalyst, maleic acid, for hemicellulose hydrolysis in corn stover. Hemicellulose hydrolysis and xylose degradation kinetics in the presence of maleic acid was compared to sulfuric acid. At optimized reaction conditions for each acid, maleic acid hydrolysis results in minimal xylose degradation, whereas sulfuric acid causes 3-10 times more xylose degradation.

According to Saha, B.C. et al in Process Biochemistry, 2005, volume 40, pages 3693-3700 wheat straw consists of 48.57 0.30% cellulose and 27.70 0.12% hemicellulose on dry solid (DS) basis and has the potential to serve as a low cost feedstock for production of ethanol. Saha et al describe dilute acid pretreatment at varied temperature and enzymatic saccharification for conversion of wheat straw cellulose and hemicellulose to monomeric sugars. Under optimized conditions no measurable quantities of furfural and hydroxymethyl furfural were produced.

Karimi et al in Biomass and Bioenergy, 2006, volume 30, pages 247-253 describe a study that deals with the effect of some hydrolysis parameters in production of monomeric sugars by dilute-acid hydrolysis of rice straw. at relatively higher temperature than the previous works. The variables considered were the effects of the hydrolysis pressure (and temperature), one or two-stage hydrolysis, retention time and addition of sulfuric acid in different stages. In addition to the dominant sugars, glucose and xylose, the hydrolyzate were characterized with respect to acetic acid,furfural, and HMF. Formation of furfural and HMF were functions of the hydrolysis pressure, acid concentration, and retention ime, whereas the concentration of acetic acid was almost constant at pressure of higher than 10 bar and a total retention time of 10 min.

It is an object of the present invention to provide a method for the preparation of acid-treated vegetable material which is suitable for use as or for being processed into animal feed, possibly after further enzymatic treatment, or which is suitable for being processed into biofuels and biochemicals after further enzymatic treatment.

A further object of the present invention is to provide a method for the preparation of an acid-treated vegetable material for which limited amounts of acid are needed.

Another object of the present invention is to provide a method for the preparation of an acid-treated vegetable material in which less byproducts are formed.

It is furthermore an object of the present invention to provide a method in which a solid material can be used as the starting material.

Another object of the present invention is to provide a method for enzymatic treatment of acid-treated carbohydrate-containing materials.

One or more of the above objects are accomplished by using the method as described in the introduction, which comprises the steps of:
(a) providing a mixture of a carbohydrate-containing vegetable material, an organic acid and, if necessary, water,
(b) heating the mixture obtained in step (a) to a temperature of at least 120 °C for some time so as to obtain a mixture comprising an acid-treated insoluble vegetable material, water, a carbohydrate-containing vegetable material that has been rendered soluble and possibly an organic acid,
(c) repeating step (b) at least once, with steps (d) and (e) being carried out prior to step (b):
(d) separating the acid-treated insoluble vegetable material from the mixture obtained in step (b) so as to obtain a liquid fraction which comprises carbohydrate-containing vegetable material that has been rendered soluble,
(e) adding additional carbohydrate-containing vegetable material and possibly additional organic acid to the liquid fraction obtained in step (d),
wherein the mixture that is used upon repeating step (b) is the mixture that is obtained in step (e).

Several high-fibre vegetable materials (biomass) represent an enormous source of sugars (saccharides) such as glucose, xylose, arabinose and uronic acids, such as glucuronic acid and galacturonic acid, which are also regarded as sugars here. Said sugars are present in bound form, in the form of carbohydrates, such as (hemi)cellulose and pectin, in the vegetable materials. In said fibrous vegetable materials also lignin is present, another fibrous fraction, which is not a carbohydrate, however. So far the sugars could only be used in thermal processes, such as combustion or gasification, for example. In their bound form, said sugars were not (very) suitable for other uses, for example as animal feed or as a source of biochemicals.

Pre-treatments of vegetable material followed by biological conversions have been studied in the past. Said pre-treatments vary from very mild to very intensive, their goal being to digest carbohydrate-containing vegetable materials and to render said materials soluble. The term "digestion" is understood to mean that both the soluble and the insoluble carbohydrates in the vegetable material are released, and are thus readily available for possible hydrolysis or other treatments. Said pre-treatments of vegetable material are followed by existing biological processes, such as enzymatic and/or microbiological conversion steps. So far said process steps and the possible combinations thereof were not economically feasible because of the high costs, such as fermentation costs, enzyme costs and costs for the further refinement of obtained products.

Recently the economic evaluation of such processes changed from the mere comparison with fuels (obtained from oil) to an integrated evaluation. In such an integrated evaluation, the availability and cost of fuels are assessed, taking into account environmental matters, such as the production of greenhouse gases.

Methods for the pre-treatment of vegetable materials, in which high concentrations of strong inorganic bases and/or acids, such as sodium hydroxide or sulphuric acid, are used are costly and involve wastage of the bases and/or acids that are used. Methods in which low concentrations of strong inorganic bases and/or acids are used in the pre-treatment of vegetable material result in products exhibiting incomplete digestion, so that said methods are not profitable. With such low concentrations of digested biomass, additional concentration steps, which take a great deal of energy and time and which are costly, therefore, need to be carried out after completion of the pre-treatment.

Another drawback of the use of inorganic acids and bases is that said use results in the formation of byproducts from sugars during the pre-treatment. Because glucose is converted into the byproduct hydroxymethyl furfural, for example, this is a source of loss of valuable glucose. Such byproducts inhibit fermentation and may be harmful to animals, which is undesirable, since it is preferable to use the pre-treated material directly in the production of bioethanol and animal feed, respectively. The formation of decomposition products of sugars, such as hydroxymethyl furfural and furfural, reduces not only the yield but also the productivity in the preparation of feed and in the production of ethanol by fermentation, because an additional step is required for removing said byproducts.

In the case of processes that result in a low concentration of digested biomass, subsequent processes, such as enzymatic or microbiological treatments, will likewise result in low concentrations of the desired products. With regard to the extraction of bioethanol from such processed mixtures it is a major advantage that a great deal of energy is required because of the low concentrations. With regard to the preparation of feed such low concentrations are also disadvantageous, because it dilutes the feed too much, so that costly drying steps are required.

As a result of the pre-treatment of the vegetable material, the carbohydrates are digested, for example for enzymatic hydrolysis. Said enzymatic hydrolysis results in the formation of at least monosaccharides from the dissolved and digested carbohydrates so as to obtain a usable product. In this way it is also possible, however, to extract disaccharides and olisaccharides so as to obtain a usable product (the term "saccharide" will generally be used herein to refer to monosaccharides, disaccharides and/or olisaccharides). The product obtained after enzymatic hydrolysis can be used as feed or as a starting material for biofuels or chemical products.

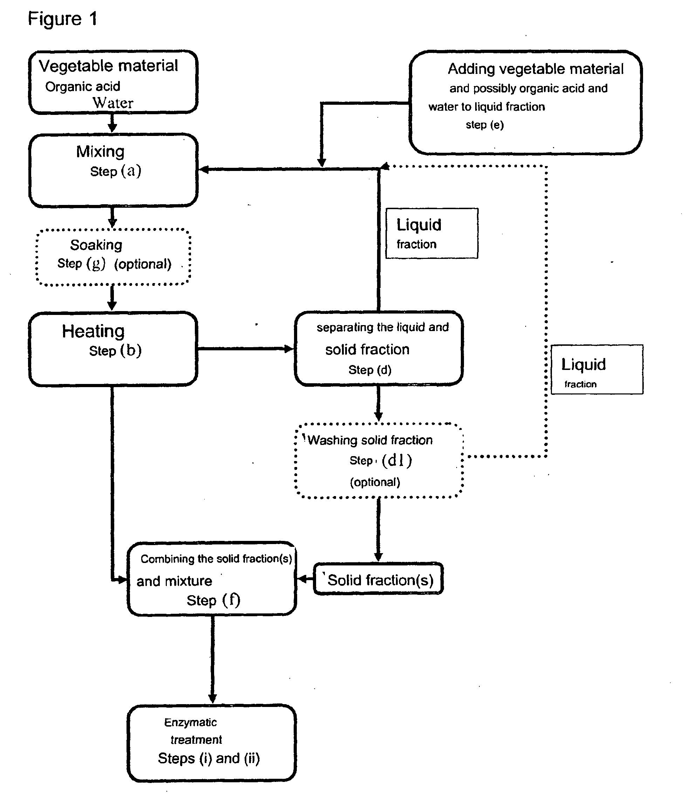

The method according to the present invention as described above relates to a method for the pre-treatment of vegetable material, comprising steps (a) - (e). A process diagram is shown by way of illustration in figure 1.

Figure 1 is a schematic representation of the process according to the invention.

Step (a) comprises providing a mixture of vegetable material, water and an organic acid. Optionally this mixture is pre-soaked. In step (b) the mixture is heated to a temperature of 120 °C for some time. The heating step has resulted in the carbohydrates in the vegetable material being partially digested and the carbohydrates that have been rendered soluble having dissolved. As a result of said digestion, the insoluble carbohydrates are available for any subsequent process steps that may be carried out, such as enzymatic or microbiological processing of the vegetable material.

From step (b) a mixture is obtained comprising an acid-treated insoluble vegetable material, water, carbohydrate-containing vegetable material that has been rendered soluble and possibly an organic acid.

The advantage of using an organic acid instead of an inorganic acid is that less byproducts are formed. Such byproducts may have a negative effect on the possible further processing of the products obtained by using the method according to the present invention as described above. Said byproducts may also have a negative effect on the usability of the obtained products.

The mixture obtained in step (b) is subsequently separated in step (d). This results in two organic acid-treated fractions: a liquid fraction comprising vegetable material containing carbohydrates in a certain concentration, which material has been rendered soluble, and a solid fraction comprising acid-treated insoluble vegetable material. This may be done by means of known separation methods, such as pressing or centrifuging, for example. After a possible washing step, the obtained solid fraction is stored for possible subsequent processing, for example in enzymatic hydrolysis.

The obtained liquid fraction is reused (step (c)) by adding vegetable material to said fraction again in step (e). Additional organic acid may be added for adjusting the pH value of the mixture to a desired value. In the present invention, the organic acid is a catalyst for the hydrolysis of carbohydrates, among other things. Since the mixture is heated, a small portion of the organic acid may be decomposed. Moreover, the fibre fraction may bind acid. Because of these effects, the effective acidity level will decrease, as a result of which the pH will increase in comparison with the original acid solution. To compensate for this, acid may be added.

Subsequently, said mixture is heated again as in step (b), after which step (d) can be carried out again, also in this case to obtain a solid fraction and a liquid fraction. This cycle of separating (step (d)), adding vegetable material and possibly acid (step (e)) and heating (step (b)) may be repeated several times, if desired. The concentration of dissolved carbohydrates in the liquid fraction will increase each time the cycle is repeated.

Higher concentrations of carbohydrates were not possible in prior art methods, because more vegetable material had to be added to the acidic solution in the first step so as to obtain a higher concentration. This resulted in a mixture having a high viscosity, or a moist yet solid reaction mixture, during the pre-treatment. This impeded the mixing process, so that the pre-treatment could no longer be carried out correctly. The present inventors have found that it is possible to process a larger amount of vegetable material by reusing the liquid fraction so as to obtain a higher carbohydrate concentration, and that without the disadvantageous high viscosity or low miscibility. After all, the solid, insoluble fraction is removed after every cycle.

According to a preferred embodiment of the method according to the present invention, step (c) is repeated at least twice, in particular three times. Reusing the liquid fraction at least twice leads to a higher concentration of dissolved carbohydrates in the liquid fraction is higher than in the situation in which the liquid fraction is reused once. Said second reuse also results in an additional amount of solid fraction. Because of said second reuse, a higher yield of carbohydrates per unit of organic acid will be effected, therefore. According to a more preferred embodiment of the method according to the present invention, step (c) is repeated at least three times. As a result of said additional repetition, the concentration of dissolved carbohydrates in the liquid fraction will be higher than in the case of two repetitions. Furthermore, an additional solid fraction will be obtained. Repeating step (c) will thus also result in an even higher yield of carbohydrate per unit of organic acid, which is economically advantageous.

Of course it is also possible to use a larger number of repetitions. In that case it is necessary to check each time the step has been repeated if the viscosity of the obtained liquid fraction is still low enough for carrying out step (b) again. Thus it is in fact the viscosity of the liquid fraction that determines the number of repetitions. Said viscosity is in practice the resistance of the starting material/water/acid mixture to mixing. This interferes with the admixing of, for example, additional acid. In a physical sense, said resistance is the energy that is used for obtaining a specific mixing result. Traditionally, viscosity in particular relates to the "treacliness" of liquid, i.e. molecularly dissolved materials are concerned. A very high concentration of small, solid particles has the same effect, however, especially if said particles are of a fibrous nature. The number of repetitions will therefore depend in part on the viscosity and thus the composition in step (a).

It is preferable if the method according to the present invention comprises an additional step (f), which is carried out after step (c). Said additional step (f) comprises combining the acid-treated insoluble vegetable material obtained in step (d) (solid fraction) with the mixture obtained in step (b) (a combination of the liquid and solid fractions). Such a mixture comprises a high concentration of dissolved carbohydrates and a large amount of acid-treated insoluble vegetable material. As a result of the acid treatment, the insoluble carbohydrates in the vegetable material are digested to a large extent and consequently available for possible further enzymatic treatment. Combining the fractions leads to a higher sugar content upon further processing. Because of this higher content, additional concentration steps for the enzymatic treatment are not required. As a result of said combination, the mixture will furthermore be more easily manageable upon further processing, and an enzymatic and/or micro biologic treatment needs to be carried out only once instead of a number of times for the individual solid and liquid fractions. As a result of said combination, the mixture will furthermore be more easily manageable upon further processing.

Another embodiment of the method according to the present invention comprises an additional step (d1) to be carried out between step (d) and step (e). Said additional step (d1) comprises washing the acid-treated insoluble vegetable material obtained in step (d) with water so as to obtain a second liquid fraction. Preferably, the second liquid fraction is combined in step (d1) with the liquid fraction obtained in step (d). Such a washing step and the reuse of the ashing water leads to a higher yield of dissolved carbohydrates. Said yield is increased in that any soluble carbohydrates that remained behind in the solid fraction are washed out of said solid fraction and added to the liquid fraction. The washing water may also comprise an organic acid; as a result, separate additional acidification prior to or heating the mixture may not be necessary.

Another embodiment of the method according to the present invention comprises an additional step (a1) to be carried out prior to step (a). Said additional step (a1) comprises washing the carbohydrate-containing vegetable material at least once. The carbohydrate-containing vegetable material is preferably washed with water. The carbohydrate-containing vegetable material may occasionally be contaminated with undesirable materials during harvesting, for example with sand, but also with compounds having a buffering effect, for example. The presence of such undesirable materials has an adverse effect on the efficiency of the acid hydrolysis. Since compounds having a buffering effect may be present during the acid hydrolysis, for example, more acid needs to be added, which leads to a higher acid consumption, which is disadvantageous for economic reasons. Furthermore, the undesirable material has an adverse effect on the enzymatic hydrolysis, so that the hydrolysis takes place less efficiently. As a result, the products obtained after enzymatic .hydrolysis, when used as feed product, will have a lower nutritional value. Furthermore, the presence of such undesirable materials will.have a negative effect on the extraction of biofuels or chemical products from the product obtained after enzymatic hydrolysis. The presence of sand furthermore leads to excessive wear of machines used in carrying out the present invention. Since the washing of the carbohydrate-containing vegetable material takes place prior to step (a), the greater part of the undesirable materials is removed from the carbohydrate-containing vegetable material in said washing step, so that the above-described negative effect of said undesirable materials will be minimised.

It is furthermore preferable that the undesirable materials are removed from the washing water after completion of step (a1). Solid particles, such as sand, for example, can for example, be removed by straining centrifuging or by being allowed to settle. Once the undesirable materials have been removed from the washing water, said washing water can be reused, if desired. Because the water can be reused, the water consumption during these washing steps is minimised, which is advantageous for economic reasons as well as for environmental reasons.

The organic acid that is used preferably has a pKa value of 1.3 - 5.0. The present inventors have found that organic acids having a pKa value of 1.3 - 5.0 provide a satisfactory digestion of carbohydrates without the resulting formation of an inconveniently large amount of harmful and undesirable byproducts, such as furfural and hydroxymethyl furfural. If an acid having a pKa value of 1.3 is used, more byproducts will be formed. If the pKa value is higher than 5.0, the digestion of carbohydrates may be undesirably low. A few examples of organic acids having a pKa value of 1.3 - 5.0 which are suitable for use in the present invention are shown in Table 1.

**Table 1**

| Acid | Formula | pKa |
|---|---|---|
| Formic acid | HCOOH | 38 |
| Acetic acid | CH₃COOH | 48 |
| Propanic acid | CH₃CH₂COOH | 48 |
| Butyric acid | C₃H₇COOH | 48 |
| Malonic acid | HOOCCH₂COOH | 29 |
| Succinic acid | HOOC(CH₂)₂COOH | 42 |
| Glutaric acid | HOOC(CH₂)₃COOH | 43 |
| Adipinic acid | HOOC(CH₂)₄COOH | 44 |
| Maleic acid | cis- HOOC-CH=CH-COOH | 20 |
| Fumaric acid | trans- HOOC-CH=CH-COOH | 30 |
| Lactic acid | CH₃CHOHCOOH | 39 |
| Malic acid | CH₃CH₂CHOHCOOH | 34 |
| Tartaric acid | CH₃CHOHCHOHCOOH | 30 |
| Citric acid | CH₂CHOHCH₂(COOH)₃H₂O | 31 |
| Oxalic acid | HOOC-COOH | 13 |
| Carbonic acid | | 36 |

The organic acid is preferably selected from the group consisting of fumaric acid, maleic acid, citric acid and lactic acid or a combination thereof. Acids from this group are used because they exhibit a satisfactory activity and may be contained in feed without being harmful to animals. Furthermore, fumaric acid, citric acid, lactic acid and possibly also maleic acid have nutritional value for the animal. Said acids further do not have a negative effect on the ethanol fermentation process. Fumaric acid is to be preferred because of its satisfactory activity in the digestion of carbohydrates.

It is furthermore preferable that the amount of organic acid in the mixture of step (a) is at most 4 mol, based on one kilogram of vegetable material. Carbohydrates dissolve well in a mixture having such an acid content, the carbohydrates in the vegetable material are digested well and less harmful byproducts are formed.

Preferably, the acid content is inversely proportional to the pKa value of the acid that is used. In other words, the lower the pKa value, the lower the acid content because the higher the pKa value, the weaker the acid.

The acid content is in that case preferably more than 0.5 mol per kilogram of vegetable material if an organic acid having a pKa value higher than or equal to 4.0 is used. If not enough acid having a pKa value higher than or equal to 4.0 is added, the digestion of the vegetable material will be unsatisfactory, resulting in a lower saccharide yield.

It is furthermore preferable if the acid content is less than 0.5 per kilogram of vegetable material if an organic acid having a pKa value of less than 4.0 is used. If too much acid having a pKa value of less than 4.0 is used, an excessive amount of byproducts will be formed.

In one embodiment of the method according to the present invention, the mixture that is provided in step (a) has a dry matter content in the 5 - 50% range. Such a mixture provides a satisfactory digestion of the vegetable material and a high concentration of dissolved carbohydrates. If the dry matter content is low, the yield of dissolved and digested carbohydrates will be low as well, which is economically disadvantageous. Furthermore, mixtures having such a dry matter content are easily manageable in a method according to the present invention. If the dry matter content is more than 50%, the mixture will be less easily manageable. The present inventors have found a good compromise between the aforesaid properties for a dry matter content in the 5 - 50% range, which is preferred, therefore. A particularly good compromise was found in the 10 - 40% range, which is preferred, therefore.

The vegetable material that is used preferably has a cellulose content of at least 5 wt.%. Materials having a lower cellulose content can also be treated, but vegetable materials having a lower content are less cost-effective. The processing of said materials results in a product having too low a sugar content.

It is furthermore preferable if the vegetable material is a cellulose-containing vegetable material. Said materials are usually stalks, branches and trunks, roots, leaves, residue of seeds used for oil and/or starch extraction, for example straw, corn stalks and leaves, grass and combinations thereof. Also (residue of) corn, wheat, turnip and soy products, wood, jatropha, oil palm and sugar cane. Furthermore residue from materials used for the production of biofuels or starch, such as fibrous, high-carbohydrate residual flows from corn, barley and wheat processing. Said vegetable material has a carbohydrate content high enough for extracting sufficient sugars therefrom and being economically feasible. It is preferable to make a selection from the above group, because the raw materials from said group are available on a large scale as residual products from the agricultural and industrial sectors. Traditionally, said products are reduced by chopping; i.e. reducing by means of knives to pieces frequently about 5 mm in size, but other sizes are also possible.

The temperature in step (b) is preferably in the 120 - 210 °C range, more preferably in the 150 - 180 °C range. At temperatures below 120 °C, organic acids have little effect on the digestion process, if any. At temperatures above 120 °C, harmful byproducts are formed. Too high a temperature may also lead to decomposition of the organic acid. The present inventors have found that a particularly good comprise between the above properties is found in the 150 - 180 °C range.

It is preferable to use a pressure of between 1 and 25 bar, preferably between 4 and 10 bar, during step (b). Heating at an elevated pressure makes it possible to use the aforesaid temperatures above 100 °C, since an aqueous mixture is concerned here. Such a pressure can for example be obtained by heating the mixture in a closed vessel. As a result, the pressure will increase due to heating and gasification. It is also possible to apply an elevated pressure in advance, however.

It is furthermore preferable to use a pressure of between 100 and 200 bar during step (b) if carbonic acid is used as the acid. As a result of the use of such a high pressure, carbon dioxide has dissolved in the liquid phase and partially dissociated as carbonic acid. By using carbonic acid under the aforesaid pressures, it is possible to realise a pKa value in the aforesaid pKa range of 1.3 - 5.0.

The heating in step (b) is preferably carried out for 5 to 60 minutes. Sufficient carbohydrate dissolves during this period and an adequate digestion takes place. If step (b) is too short, digestion will be inadequate and the yield will be low, which is economically disadvantageous. If step (b) lasts too long, an excessive amount of byproducts will be formed, which is disadvantageous. A second disadvantage of carrying, out step (b) for too long a period of time is the amount of energy required for prolonged heating, which is economically disadvantageous. The present inventors have found a particularly good compromise by using a period of 15 to 30 minutes.

Preferably, step (a), step (e), or both, comprise(s) and additional step (g), in which the mixture obtained after carrying out step (a) or (b) is pre-soaked for at most 24 hours. As a result of said optional pre-soaking step, the organic acid penetrates better into the vegetable material, resulting in a high degree of digestion. As a result of the high degree of digestion, a good digestion of the carbohydrates in the solid vegetable material is obtained, resulting in a high yield of digested and dissolved carbohydrates. Said higher degree of digestion is economically advantageous.

A carbohydrate-containing vegetable material that has been rendered soluble can be obtained from step (d) or step (f).

Such a carbohydrate-containing vegetable material obtained from step (d) is the liquid fraction, it has a high concentration of carbohydrates and contains few byproducts. Such a solution is therefore suitable for the enzymatic production of bioethanol, for example, or for use as animal feed or for use in the preparation thereof.

Such a carbohydrate-containing vegetable material obtained from step (d) is also the solid fraction and comprises acid-treated insoluble vegetable material. Such material is suitable for use as cattle feed or for being subjected to an enzymatic treatment so as to obtain free sugars. This is made possible in that the insoluble carbohydrates have been digested by the acid treatment and are therefore available for the enzymatic treatment.

Such a carbohydrate-containing vegetable material obtained from step (f) is a mixture which is suitable for use as animal feed or for being subjected to an enzymatic treatment so as to obtain free sugars. In addition to the high carbohydrate concentration, this is made possible in that the insoluble carbohydrates have been digested by the acid treatment and are therefore available for enzymatic treatment. Such a mixture is suitable for further processing into animal feed, biochemical, and biofuels, such as bioethanol.

The present invention further relates to a method for the enzymatic treatment of the acid-treated carbohydrate-containing vegetable materials obtained from steps (d) and (f). This method comprises the steps of:
(i) providing the organic acid-treated carbohydrate-containing vegetable material from step (d) or step (f); optionally adjusting the pH of the carbohydrate-containing vegetable material,
(ii) reacting the saccharide-containing material obtained in step (i) with an enzyme for splicing off saccaride.

The enzymes used may be any enzymes from the group of known hydrolysed enzymes, in particular enzymes of the following types: cellulase, hemicellulase and pectinase. The required reaction time will depend on the selected type of enzyme and the concentration thereof. With such a treatment, monosaccharides, disaccharides or oligosaccharides or combinations thereof, among other substances, can be obtained. If desired, the pH of the obtained mixture can be adjusted prior to the enzymatic hydrolysis by the addition of an extra amount of an acid or a base. The desired pH may depend on the selected enzyme that functions optimally within a particular pH range. Adjustment of the pH will lead to an increased efficiency of the enzymatic hydrolysis.

Such an enzymatic treatment leads to an increase of the amount of freely soluble saccharides. The high concentration of free saccharides and the low concentration of byproducts, among other factors, make the mixture suitable for further processing into animal feed. Because of the high concentration of free saccharides, said mixture is also suitable for fermentative bioethanol production.

Said enzymatic treatment is carried out after the above-discussed methods according to the present invention have been carried out. This makes it possible to obtain a mixture having a high saccharide content from vegetable material within a short period of time. The short period of time in combination with a low consumption of organic acid and the obtained high saccharide content is economically advantageous.

The present invention will be explained in more detail below with reference to a number of non-limitative examples.

Further embodiments of the present invention are defined in the claims.

### Examples.

Example 1. Formation of furfural and hydroxymethyl furfural (HMF) during acid hydrolysis with organic acids according to the present invention and with sulphuric acid. For each acid, the hydrolysis was carried out with 70 g of acid per kg of straw. The acid hydrolysis was carried out for 30 minutes with a dry matter content of 10%. The results are shown in Tables 2 and 3, in which the amounts of furfural (Table 2) and HMF (Table 3) are expressed in grammes per kilogram of straw. The values in Tables 2 and 3 clearly show that acid hydrolysis with acids according to the present invention leads to a lower production of furfural and HMF than hydrolysis with sulphuric acid.

**Table 2**

| furfural(g/kg) | | | |
|---|---|---|---|
| temp. °C | fumaric acid | maleic acid | sulphuric acid |
| 130 | 0.2 | 0.2 | 1.1 |
| 150 | 1.7 | 3.1 | 8.4 |
| 170 | 7.8 | 8.0 | 15.5 |

**Table 3**

| HMF (g/kg) | | | |
|---|---|---|---|
| temp. °C | fumaric acid | maleic acid | sulphuric acid |
| 130 | 0.2 | 0.2 | 0.3 |
| 150 | 0.6 | 0.8 | 2 |
| 170 | 1.1 | 1.3 | 1.9 |

Example 2. In this example, straw was treated with maleic acid. Maleic acid and straw were mixed to obtain a mixture. After heating at 150 °C and subsequent cooling, the mixture was compressed. This resulted in a solid fraction (a first moist fibre cake) and a liquid fraction (fraction 1) containing maleic acid, among other substances. Subsequently, a second amount of straw was added to fraction 1, followed by mixing to obtain a mixture. After heating at 150 °C and subsequent cooling, the mixture was compressed. This resulted in a solid fraction (a second moist fibre cake) and a liquid fraction (fraction 2). Of said fractions 1 and 2, the glucose concentration was determined. Also the concentrations of fumaric acid, xylose, arabinose and furfural were determined. The results are shown in Table 4.

**Table 4.**

| | acid | Fraction 1 | Fraction 2 |
|---|---|---|---|
| water | 66.0 | 60 | 58 |
| maleic acid | 0.38 | 0.297 | 0.295 |
| glucose | | 0.082 | 0.154 |
| fumaric acid | | 0.008 | 0.014 |
| xylose | | 0.839 | 0.850 |
| arabinose | | 0.110 | 0.193 |
| furfural | | 0.026 | 0.080 |

Fraction 1 shows the glucose yield obtained without reuse of the acid, fraction 2 shows the glucose yield after reuse of the acid. From Table 4 it can be derived that the reuse of the acid (fraction 2) results in a glucose concentration in the liquid fraction which is nearly twice as high as the glucose concentration obtained without reuse of the acid (fraction 1).

Example 3. Removal of sand from the vegetable material. 621 g of cornstover having a dry matter content of 26 wt.% and 20 wt.% of ash, relative to the weight of the dry matter, was stirred with 3 I of water in a vessel for 2-3 minutes. The fibrous material was then collected, using a household screen, and squeezed so as to remove as much washing water as possible. The remaining fibrous mass weighed 564 g. After drying at 50 °C in a drying oven for 24 hours, the dried residue weighed 108 g. Said residue contained a remaining amount of 4.7 g of residual moisture and 7.5 g of ash. The water that was squeezed out contained fibres of organic material and sand. After decanting, the remaining sand was weighed after drying at 50 °C for 24 hours. The weight of the dried sand was 25 g. From these data it was calculated that the cornstover initially contained 33 g of ash. After washing, 8 g of ash remained in the dry material and 25 g of washed-out sand=ash. It is known that sand-free cornstover contains about 6-7% of ash, which naturally occurs in the plant. On the basis of this, about 7 g of ash can be expected to be present in 108 g of residue after drying. The amount of sand that remains will be about 1 g, therefore. Relative to the 25 g of washed-out sand, this means that about 95% of the sand has been removed by washing.

Example 4. Furthermore, the effect of the step of washing the vegetable material on the pH during acid hydrolysis was examined. Various amounts of acid were added to washed or unwashed cornstover and wheat straw. After acid hydrolysis, the pH of the material was determined as a measure of the amount of free acid. Furthermore, the amounts of freed glucose were measured after subsequent enzymatic treatment. The results are shown in Table 5.

**Table 5.**

| | | unwashed | | | washed | | |
|---|---|---|---|---|---|---|---|
| | acid | quantity (g/kg dry matter) | pH | glucose kg/ ton dry matter | Quantity (g/kg dry matter | pH | glucose kg/ton dry matter |
| Cornstover | No acid | 0 | 5.7 | 66 | 0 | 4.92 | 89 |
| | lactic acid | 5.3 | 5.1 | 99 | 4.9 | 4.56 | 100 |
| | lactic acid | 10.6 | 5 | 96 | 9.9 | 4.41 | 103 |
| | lactic acid | 104.4 | 3.8 | 90 | 98.9 | 3.33 | 110 |
| | citric acid | 5.3 | 5.1 | 82 | 4.9 | 4.46 | 104 |
| | citric acid | 10.6 | 5.6 | 55 | 10 | 4.44 | 106 |
| | citric acid | 105 | 3.9 | 108 | 98.4 | 3.29 | 109 |
| Wheat straw | No acid | 0 | 5.44 | 50 | 0 | 5.22 | 70 |
| | lactic acid | 4.6 | 5.7 | 47 | 5.2 | 5.21 | 76 |
| | lactic acid | 9.2 | 5.18 | 53 | 10.4 | 4.6 | 65 |
| | lactic acid | 91.6 | 3.46 | 83 | 104.2 | 3.2 | 129 |
| | citric acid | 3.6 | 5.44 | 47 | 5.2 | 4.55 | 66 |
| | citric acid | 7.3 | 5.29 | 43 | 10.4 | 4.5 | 64 |
| | citric acid | 73.1 | n.m* | 76 | 103.7 | 3.11 | 103 |

| | | | | | | | |
|---|---|---|---|---|---|---|---|
| * not measured | | | | | | | |

Table 5 shows that washing in advance appears to decrease the pH and increase the amount of freed glucose both for cornstover and for straw in comparison with unwashed cornstover or straw.

## Claims

1. A method for treating carbohydrate-containing vegetable material with acid, comprising the steps of:
(a) providing a mixture of a carbohydrate-containing vegetable material, an organic acid and, if necessary, water,
(b) heating the mixture obtained in step (a) to a temperature of at least 120 °C for some time so as to obtain a mixture comprising an acid-treated insoluble vegetable material, water, a carbohydrate-containing vegetable material that has been rendered soluble and possibly an organic acid,
(c) repeating step (b) at least once, with steps (d) and (e) being carried out prior to step (b):
(d) separating the acid-treated insoluble vegetable material from the mixture obtained in step (b) so as to obtain a liquid fraction which comprises carbohydrate-containing vegetable material that has been rendered soluble,
(e) adding additional carbohydrate-containing vegetable material and possibly additional organic acid to the liquid fraction obtained in step (d),
wherein the mixture that is used upon repeating step (b) is the mixture that is obtained in step (d).

2. A method according to claim 1, **characterised in that** the number of repetitions of step (c) is at least two, preferably at least three.

3. A method according to claim 1 or 2, **characterised in that** the method comprises an additional step (f), which is carried out after step (c):
(f) combining the acid-treated insoluble vegetable material obtained in step (d) and the mixture obtained in step (b).

4. A method according to one or more of claims 1 - 3, **characterised in that** an additional step (d1) is carried out between step (d) and step (e):
(d1) washing the acid-treated insoluble vegetable material obtained in step (d) with water so as to obtain a second liquid fraction.

5. A method according to claim 4, **characterised in that** the second liquid fraction obtained in step (d1) is combined with the liquid fraction obtained in step (d).

6. A method according to one or more of the preceding claims, **characterised in that** the organic acid has a pKa value of 1.3 - 5.0.

7. A method according to one or more of the preceding claims, **characterised in that** the temperature in step (b) is in the 120 - 210 °C range, preferably in the 150 - 180 °C range.

8. A method according to one or more of the preceding claims, **characterised in that** the mixture provided in step (a) has a dry matter content in the 5 - 50% range, preferably in the 10 - 40% range.

9. A method according to one or more of the preceding claims, **characterised in that** the organic acid is selected from the group consisting of fumaric acid, maleic acid, citric acid and lactic acid or a combination thereof, preferably fumaric acid.

10. A method according to one or more of the preceding claims, **characterised in that** a pressure between 1 and 25 bar, preferably between 4 and 10 bar, is used in step (b) and wherein a pressure between 100 and 200 bar is used in step (b) if carbonic acid is used as the organic acid.

11. A method according to one or more of the preceding claims, **characterised in that** said heating in step (b) takes place for 5 to 60 minutes, preferably 15 to 30 minutes.

12. A method according to one or more of the preceding claims, **characterised in that** step (a) and/or step (e) comprises an additional step (g), in which the mixture obtained after carrying out step (a) or step (e) is pre-soaked for at most 24 hours.

13. A method according to one or more of the preceding claims, **characterised in that** prior to step (a) an additional step (a1) is carried out:
(a1) washing the carbohydrate-containing vegetable material at least once.

14. A method according to claims 1-3 comprising the steps of:
(i) providing the organic acid-treated carbohydrate-containing vegetable material from steps (d) or (f), optionally adjusting the pH of the carbohydrate-containing vegetable material,
(ii) reacting the material obtained in step (i) with at least an enzyme for splicing off saccaride.

## Patentansprüche

1. Verfahren zur Säurebehandlung eines kohlenhydratenthaltenden pflanzlichen Materials, das die folgenden Schritte umfasst:
a) Bereitstellen einer Mischung aus einem kohlenhydratenthaltenden pflanzlichen Material, einer organischen Säure und, wenn nötig, Wasser,
b) Erwärmen der in Schritt a) erhaltenen Mischung auf eine Temperatur von wenigstens 120°C für einige Zeit, um so eine Mischung zu erhalten, die ein säurebehandeltes, unlösliches pflanzliches Material, Wasser, ein kohlenhydratenthaltendes pflanzliches Material, das löslich gemacht worden ist, und eventuell eine organische Säure umfasst,
c) Wiederholen von Schritt b) wenigstens einmal, wobei die Schritte d) und e) vor Schritt b) ausgeführt werden:
d) Abtrennen des säurebehandelten, unlöslichen pflanzlichen Materials von der in Schritt b) erhaltenen Mischung, um so einen flüssigen Teil zu erhalten, der kohlenhydratenthaltendes pflanzliches Material aufweist, das löslich gemacht worden ist,
e) Hinzufügen von zusätzlichem kohlenhydratenthaltenden pflanzlichen Material und eventuell zusätzlicher organischer Säure zu dem in Schritt d) erhaltenen flüssigen Teil,
wobei die Mischung, die beim Wiederholen von Schritt b) verwendet wird, die Mischung ist, die in Schritt d) erhalten wird.

2. Verfahren nach Anspruch 1, **dadurch gekennzeichnet, dass** die Anzahl an Wiederholungen von Schritt c) wenigstens zwei ist, vorzugsweise wenigstens drei.

3. Verfahren nach Anspruch 1 oder 2, **dadurch gekennzeichnet, dass** das Verfahren einen zusätzlichen Schritt f) aufweist, der nach Schritt c) durchgeführt wird:
f) Kombinieren des in Schritt d) erhaltenen säurebehandelten, unlöslichen pflanzlichen Materials und der aus Schritt b) erhaltenen Mischung.

4. Verfahren nach einem oder mehreren der Ansprüche 1 bis 3, **dadurch gekennzeichnet, dass** ein zusätzlicher Schritt d1) zwischen Schritt d) und Schritt e) durchgeführt wird:
d1) Waschen des in Schritt d) erhaltenen säurebehandelten, unlöslichen pflanzlichen Materials mit Wasser, um so einen zweiten flüssigen Teil zu erhalten.

5. Verfahren nach Anspruch 4, **dadurch gekennzeichnet, dass** der in Schritt d1) erhaltene zweite flüssige Teil mit dem in Schritt d) erhaltenen flüssigen Teil kombiniert wird.

6. Verfahren nach einem oder mehreren der vorhergehenden Ansprüche, **dadurch gekennzeichnet, dass** die organische Säure einen pKa-Wert von 1,3 bis 5,0 hat.

7. Verfahren nach einem oder mehreren der vorhergehenden Ansprüche, **dadurch gekennzeichnet, dass** die Temperatur in Schritt b) im Bereich von 120 bis 210°C liegt, vorzugsweise im Bereich von 150 bis 180°C.

8. Verfahren nach einem oder mehreren der vorhergehenden Ansprüche, **dadurch gekennzeichnet, dass** die in Schritt a) bereitgestellte Mischung einen Trockenmaterialanteil im Bereich von 5 bis 50% hat, vorzugsweise im Bereich von 10 bis 40%.

9. Verfahren nach einem oder mehreren der vorhergehenden Ansprüche, **dadurch gekennzeichnet, dass** die organische Säure aus der aus Fumarsäure, Maleinsäure, Zitronensäure und Milchsäure oder einer Kombination davon bestehenden Gruppe ausgewählt wird, vorzugsweise Fumarsäure ist.

10. Verfahren nach einem oder mehreren der vorhergehenden Ansprüche, **dadurch gekennzeichnet, dass** ein Druck zwischen 1 und 25 bar, vorzugsweise zwischen 4 und 10 bar, in Schritt b) verwendet wird und wobei ein Druck zwischen 100 und 200 bar in Schritt b) verwendet wird, wenn Kohlensäure als organische Säure verwendet wird.

11. Verfahren nach einem oder mehreren der vorhergehenden Ansprüche, **dadurch gekennzeichnet, dass** das Erwärmen in Schritt b) 5 bis 60 Minuten lang erfolgt, vorzugsweise 15 bis 30 Minuten.

12. Verfahren nach einem oder mehreren der vorhergehenden Ansprüche, **dadurch gekennzeichnet, dass** Schritt a) und/oder Schritt e) einen zusätzlichen Schritt g) aufweisen, in dem die nach der Durchführung von Schritt a) oder Schritt e) erhaltene Mischung maximal 24 Stunden lang vorgeweicht wird.

13. Verfahren nach einem oder mehreren der vorhergehenden Ansprüche, **dadurch gekennzeichnet, dass** vor Schritt a) ein zusätzlicher Schritt a1) durchgeführt wird:
a1) wenigstens einmaliges Waschen des kohlenhydratenthaltenden pflanzlichen Materials.

14. Verfahren nach den Ansprüchen 1 bis 3, mit den folgenden Schritten:
i) Bereitstellen des mit organischer Säure behandelten, kohlenhydratenthaltenden pflanzlichen Materials aus den Schritten d) oder f), und wahlweise Einstellen des pH-Werts des kohlenhydratenthaltenden pflanzlichen Materials,
ii) Umsetzen des aus Schritt i) erhaltenen Materials mit wenigstens einem Enzym zum Aufspalten von Saccharid.

## Revendications

1. Procédé de traitement d'un matériau végétal contenant des glucides avec de l'acide, comprenant les étapes suivantes :
(a) la fourniture d'un mélange d'un matériau végétal contenant des glucides, d'un acide organique et, si nécessaire, d'eau,
(b) le chauffage du mélange obtenu dans l'étape (a) jusqu'à une température d'au moins 120 °C, pendant un certain temps, de manière à obtenir un mélange comprenant un matériau végétal insoluble traité à l'acide, de l'eau, un matériau végétal contenant des glucides qui a été rendu soluble et éventuellement un acide organique,
(c) la répétition de l'étape (b) au moins une fois, avec les étapes (d) et (e) étant réalisées avant l'étape (b) :
(d) la séparation du matériau végétal insoluble traité à l'acide du mélange obtenu dans l'étape (b) de manière à obtenir une fraction liquide qui comprend un matériau végétal contenant des glucides qui a été rendu soluble,
(e) l'addition de matériau végétal contenant des glucides supplémentaire et éventuellement d'acide organique supplémentaire à la fraction liquide obtenue dans l'étape (d),
où le mélange qui est utilisé après la répétition de l'étape (b) est le mélange qui est obtenu dans l'étape (d).

2. Procédé selon la revendication 1, **caractérisé en ce que** le nombre de répétitions de l'étape (c) est au moins de deux, de préférence au moins de trois.

3. Procédé selon les revendications 1 ou 2, **caractérisé en ce que** le procédé comprend une étape (f) supplémentaire, qui est réalisée après l'étape (c) :
(f) la combinaison du matériau végétal insoluble traité à l'acide obtenu dans l'étape (d) et du mélange obtenu dans l'étape (b).

4. Procédé selon une ou plusieurs des revendications 1 à 3, **caractérisé en ce qu'**une étape (d1) supplémentaire est réalisée entre l'étape (d) et l'étape (e) :
(d1) le lavage du matériau végétal insoluble traité à l'acide obtenu dans l'étape (d) avec de l'eau de manière à obtenir une seconde fraction liquide.

5. Procédé selon la revendication 4, **caractérisé en ce que** la seconde fraction liquide obtenue dans l'étape (d1) est combinée avec la fraction liquide obtenue dans l'étape (d).

6. Procédé selon une ou plusieurs des revendications précédentes, **caractérisé en ce que** l'acide organique a une valeur de pKa de 1,3 à 5,0.

7. Procédé selon une ou plusieurs des revendications précédentes, **caractérisé en ce que** la température dans l'étape (b) se situe dans la plage de 120 à 210 °C, de préférence dans la plage de 150 à 180 °C.

8. Procédé selon une ou plusieurs des revendications précédentes, **caractérisé en ce que** le mélange fourni dans l'étape (a) présente une teneur en matière sèche située dans la plage de 5 à 50 %, de préférence dans la plage de 10 à 40 %.

9. Procédé selon une ou plusieurs des revendications précédentes, **caractérisé en ce que** l'acide organique est choisi dans le groupe constitué d'acide fumarique, d'acide maléique, d'acide citrique et d'acide lactique ou d'une combinaison de ceux-ci, de préférence l'acide fumarique.

10. Procédé selon une ou plusieurs des revendications précédentes, **caractérisé en ce qu'**une pression entre 1 et 25 bars, de préférence entre 4 et 10 bars, est utilisée dans l'étape (b) et dans lequel une pression entre 100 et 200 bars est utilisée dans l'étape (b) si de l'acide carbonique est utilisé en tant qu'acide organique.

11. Procédé selon une ou plusieurs des revendications précédentes, **caractérisé en ce que** ledit chauffage dans l'étape (b) prend place pendant 5 à 60 minutes, de préférence 15 à 30 minutes.

12. Procédé selon une ou plusieurs des revendications précédentes, **caractérisé en ce que** l'étape (a) et/ou l'étape (e) comprennent une étape (g) supplémentaire, dans laquelle le mélange obtenu après la réalisation de l'étape (a) ou de l'étape (e) est prétrempé pendant au plus 24 heures.

13. Procédé selon une ou plusieurs des revendications précédentes, **caractérisé en ce qu'**avant l'étape (a), une étape (a1) supplémentaire est réalisée :
(a1) le lavage du matériau végétal contenant des glucides au moins une fois.

14. Procédé selon les revendications 1 à 3, comprenant les étapes suivantes :
(i) la fourniture du matériau végétal contenant des glucides traité avec un acide organique issu des étapes (d) ou (f), éventuellement en ajustant le pH du matériau végétal contenant des glucides,
(ii) la réaction du matériau obtenu dans l'étape (i) avec au moins une enzyme pour séparer le saccharide.
